# EUROPEAN PATENT SPECIFICATION

(11) **EP 1 517 722 B1**
(45) Date of publication and mention of the grant of the patent: **16.08.2006**
(21) Application number: 03742313.4
(22) Date of filing: 27.06.2003
(51) Int. Cl.: A61M 37/00, A61K 9/70

(54) **METHOD OF COATING TRANSDERMAL DRUG DELIVERY DEVICES HAVING COATED MICROPROTRUSIONS**
VERFAHREN ZUR OBERFLÄCHENBESCHICHTUNG VON VORRICHTUNGEN ZUR TRANSDERMALEN VERABREICHUNG VON ARZNEIEN MIT BESCHICHTETEN MIKROVORSPRÜNGEN
PROCEDE POUR ENDUIRE DES DISPOSITIFS D'ADMINISTRATION DE MEDICAMENT TRANSDERMIQUE AYANT DES MICROPROTUBERANCES ENDUITES

(30) Priority: 28.06.2002 US 392609 P
(43) Date of publication of application: 30.03.2005
(73) Proprietor: ALZA CORPORATION, Mountain View, CA 94039-7210 (US)
(72) Inventor: CORMIER, Michel, J., N., Mountain View, CA 94043 (US); YOUNG, Wendy, A., San Jose, CA 95129 (US); JOHNSON, Juanita, A., Belmont, CA 94002 (US); DADDONA, Peter, E., Menlo Park, CA 94025 (US); AMERI, Mahoud, Fremont, CA 94536 (US)
(74) Representative: Williams, Paul Edwin
(86) International application number: PCT/US2003/020471
(87) International publication number: WO 2004/002566

(56) References cited:
- WO-A-00/35530
- WO-A-00/45797
- WO-A-02/07813
- WO-A-02/30301
- WO-A-96/10630

## Description

### TECHNICAL FIELD

This invention relates to administering and enhancing transdermal delivery of an agent across the skin. More particularly, the invention relates to a percutaneous drug delivery system for administering a pharmacologically active agent through the stratum corneum using skin piercing microprotrusions which have a dry coating of the pharmacologically active agent. Said dry coating having been formed from a solution containing surfactants and wetting agents and applied to microprotrusions which have optionally been surface treated. Delivery of the agent is facilitated when the microprotrusions pierce the skin of a patient and the patient's interstitial fluid contacts and dissolves the active agent.

Drugs are most conventionally administered either orally or by injection. Unfortunately, many medicaments are completely ineffective or have radically reduced efficacy when orally administered since they either are not absorbed or are adversely affected before entering the bloodstream and thus do not possess the desired activity. On the other hand, the direct injection of the medicament into the bloodstream, while assuring no modification of the medicament during administration, is a difficult, inconvenient, painful and an uncomfortable procedure which sometimes results in poor patient compliance.

Hence, in principle, transdermal delivery provides for a method of administering drugs that would otherwise need to be delivered via hypodermic injection or intravenous infusion. Transdermal drug delivery offers improvements in both of these areas. Transdermal delivery when compared to oral delivery avoids the harsh environment of the digestive tract, bypasses gastrointestinal drug metabolism, reduces first-pass effects, and avoids the possible deactivation by digestive and liver enzymes. Conversely, the digestive tract is not subjected to the drug during transdermal administration. Indeed, many drugs such as aspirin have an adverse effect on the digestive tract. However, in many instances, the rate of delivery or flux of many agents via the passive transdermal route is too limited to be therapeutically effective.

The word "transdermal" is used herein as a generic term referring to passage of an agent across the skin layers. The word "transdermal" refers to delivery of an agent (e.g., a therapeutic agent such as a drug) through the skin to the local tissue or systemic circulatory system without substantial cutting or penetration of the skin, such as cutting with a surgical knife or piercing the skin with a hypodermic needle. Transdermal agent delivery includes delivery via passive diffusion as well as delivery based upon external energy sources including electricity (e.g., iontophoresis) and ultrasound (e.g., phonophoresis). While drugs do diffuse across both the stratum corneum and the epidermis, the rate of diffusion through the stratum corneum is often the limiting step. Many compounds, in order to achieve a therapeutic dose, require higher delivery rates than can be achieved by simple passive transdermal diffusion. When compared to injections, transdermal agent delivery eliminates the associated pain and reduces the possibility of infection.

Theoretically, the transdermal route of agent administration could be advantageous in the delivery of many therapeutic proteins, because proteins are susceptible to gastrointestinal degradation and exhibit poor gastrointestinal uptake and transdermal devices are more acceptable to patients than injections. However, the transdermal flux of medically useful peptides and proteins is often insufficient to be therapeutically effective due to the large size/molecular weight of these molecules. Often the delivery rate or flux is insufficient to produce the desired effect or the agent is degraded prior to reaching the target site, for example while in the patient's bloodstream.

Transdermal drug delivery systems generally rely on passive diffusion to administer the drug while active transdermal drug delivery systems rely on an external energy source (e.g., electricity) to deliver the drug. Passive transdermal drug delivery systems are more common. Passive transdermal systems have a drug reservoir containing a high concentration of drug adapted to contact the skin where the drug diffuses through the skin and into the body tissues or bloodstream of a patient. The transdermal drug flux is dependent upon the condition of the skin, the size and physical/chemical properties of the drug molecule, and the concentration gradient across the skin. Because of the low permeability of the skin to many drugs, transdermal delivery has had limited applications. This low permeability is attributed primarily to the stratum corneum, the outermost skin layer which consists of flat, dead cells filled with keratin fibers (keratinocytes) surrounded by lipid bilayers. This highly-ordered structure of the lipid bilayers confers a relatively impermeable character to the stratum corneum.

One common method of increasing the passive transdermal diffusional drug flux involves pre-treating the skin with, or co-delivering with the drug, a skin permeation enhancer. A permeation enhancer, when applied to a body surface through which the drug is delivered, enhances the flux of the drug therethrough. However, the efficacy of these methods in enhancing transdermal protein flux has been limited, at least for the larger proteins, due to their size.

Active transport systems use an external energy source to assist drug flux through the stratum corneum. One such enhancement for transdermal drug delivery is referred to as "electrotransport." This mechanism uses an electrical potential, which results in the application of electric current to aid in the transport of the agent through a body surface, such as skin. Other active transport systems use ultrasound (phonophoresis) and heat as the external energy source.

There also have been many attempts to mechanically penetrate or disrupt the outermost skin layers thereby creating pathways into the skin in order to enhance the amount of agent being transdermally delivered. Early vaccination devices known as scarifiers generally had a plurality of tines or needles which are applied to the skin to and scratch or make small cuts in the area of application. The vaccine was applied either topically on the skin, such as U.S. Patent No. 5,487,726 issued to Rabenau or as a wetted liquid applied to the scarifier tines such as U.S. Patent No. 4,453,926 issued to Galy, or U.S. Patent No. 4,109,655 issued to Chacornac, or U.S. Patent No. 3,136,314 issued to Kravitz. Scarifiers have been suggested for intradermal vaccine delivery in part because only very small amounts of the vaccine need to be delivered into the skin to be effective in immunizing the patient Further, the amount of vaccine delivered is not particularly critical since an excess amount achieves satisfactory immunization as well as a minimum amount However a serious disadvantage in using a scarifier to deliver a drug is the difficulty in determining the transdermal drug flux and the resulting dosage delivered. Also due to the elastic, deforming and resilient nature of skin to deflect and resist puncturing, the tiny piercing elements often do not uniformly penetrate the skin and/or are wiped free of a liquid coating of an agent upon skin penetration. Additionally, due to the self healing process of the skin, the punctures or slits made in the skin tend to dose up after removal of the piercing elements from the stratum corneum. Thus, the elastic nature of the skin acts to remove the active agent coating which has been applied to the tiny piercing elements upon penetration of these elements into the skin. Furthermore the tiny slits formed by the piercing elements heal quickly after removal of the device, thus limiting the passage of agent through the passageways created by the piercing elements and in turn limiting the transdermal flux of such devices.

Other devices which use tiny skin piercing elements to enhance transdermal drug delivery are disclosed In European Patent EP 0 407063A1, U.S. Patent Nos. 5,879,326 Issued to Godshall, et al., 3,814,097 issued to Ganderton, et al., 5,279,544 issued to Gross, et al., 5,250,023 issued to Lee, et al., 3,964,482 issued to Gerstel, et al. Reissue 25,637 issued to Kravitz, et al., and PCT Publication Nos. WO 96/37155, WO 96/37256, WO 96/17648, WO 97/03718, WO 98/11937, WO 98/00193, WO 97/48440, WO 97/48441. WO 97/48442, WO 98/00193, WO 99/54580, WO 98/28037, WO 96/29298, and WO 98/29365. These devices use piercing elements of various shapes and sizes to pierce the outermost layer (i.e., the stratum corneum) of the skin. The piercing elements disclosed in these references generally extend perpendicularly from a thin, flat member, such as a pad or sheet. The piercing elements in some of these devices are extremely small, some having dimensions (i.e., a microblade length and width) of only about 25 - 400 µm and a microblade thickness of only about 5 - 50 µm. These tiny piercing/cutting elements make correspondingly small microslits/microcuts in the stratum corneum for enhanced transdermal agent delivery therethrough.

Generally, these systems include a reservoir for holding the drug and also a delivery system to transfer the drug from the reservoir through the stratum corneum, such as by hollow tines of the device itself. One example of such a device is disclosed in WO 93/17754 which has a liquid drug reservoir. The reservoir must be pressurized to force the liquid drug through the tiny tubular elements and into the skin. Disadvantages of devices such as these include the added complication and expense for adding a pressurizable liquid reservoir and complications due to the presence of a pressure-driven delivery system.

Instead of a physical reservoir, it is possible to have the drug that is to be delivered coated upon the microprojections. This eliminates the necessity of a reservoir and developing a drug formulation or composition specifically for the reservoir.

It is important when the agent solution is applied to the microprojections that the coating that is formed is homogeneous and evenly applied. This enables greater amount of drug to be retained on the microprojections and also enables great dissolution of the agent in the interstitial fluid once the devices has been applied to the skin and the stratum corneum has been pierced.

In addition, a homogeneous coating provides for greater mechanical stability both during storage and during insertion into the skin. Weak and discontinuous coatings are more likely to flake off during manufacture and storage and to be wiped off by the skin during application of the microprojections into the skin.

WO 02/07813 discloses a pharmaceutical agent delivery device having a skin-piercing portion comprising a solid reservoir medium containing the pharmaceutical agent wherein the reservoir medium is coated onto the skin-piercing portion.

WO 96/10630 closes a method of introducing a biological material into a predetermined target cell population comprising (i) providing a plurality of microprobes on a support, (ii) a solid or quasi-solid mass of target cells defining an interface with the microprobes, and (iii) a biological material at the interface, and then physically contacting the cells with the microprobes to cause the microprobes to pierce the cell walls.

The method of the present invention overcomes these limitations by providing a microprotrusion device having microprotrusions which are coated with a dry homogeneous coating. The pharmacologically active agent is selected to the sufficiently potent to be therapeutically effective when delivered as a dry coating that has been formed on a plurality of skin piercing microprotusions. Further, the agent must have sufficient water solubility to form an aqueous coating solution having the necessary solubility and viscosity for coating the microprotrusions.

Thus, according to the invention, there is provided a method of coating the surface of one or more microprojections of a microprojection array comprising the steps of:
(i) providing a microprojection array comprised of one or more microprojections;
(ii) treating the surface of one or more of said microprojections of said microprojection array with a method selected from the group consisting of chemical pre-etching, plasma treatment, heat treating, rinsing with an alkaline detergent and rinsing with a wetting agent;
(iii) providing a coating formulation comprising an active agent;
(iv) applying said coating formulation to said treated surfaces of said one or more microprojections; and
(v) drying said coating formulation onto said surfaces to form a coating.

The formation of a homogeneous coating can be accomplished by enhancing the wetability of the drug formulation when it is applied tD the microprojections. This enhancement can be accomplished by a surtax treatment of the microprojections prior to the application of the drug solution or incorporating various wetting agents and surfactants in the drug solution which is then applied to the microprojections.

A microprojection array is usually made of a metal such as stainless steel or titanium. If a microprojection is made of titanium, the outer surface of the microprojection is naturally oxidized which forms a thin layer of titanium oxide which gives the surface hydrophobic properties. Stainless steel and other metals and alloys that do not oxidize readily also present hydrophobic properties. Other materials that could be used to manufacture the microprojections, such as silicon or plastics, also present hydrophobic properties.

Treatment that would modify the surface properties of a microprojection include the formation of pits by chemical pre-etching, plasma treatment, and heat treatment. Washing the microprotrusion surfaces with an alkaline detergent rinse is also effective. These and other treatments which alter the surface energy of the microprojections can have significant impact on the ability to homogeneously coat the microprojections with a drug formulation. Most preferably is the treatment of the microprojection surface with a wetting agent.

In this last case, the microprojection array is immersed in or sprayed with a solution containing a wetting agent. Then the drug solution is applied by one or more standard techniques. In between the treatment with the wetting agent solution and the drug solution, the microprojections may be rinsed and/or dried.

Wetting agents can generally be described as amphiphilic molecules. When a solution containing the wetting agent is applied to a hydrophobic substrate, the hydrophobic groups of the molecule bind to the hydrophobic substrate, while the hydrophilic portion of the molecule stays in contact with water. As a result, the hydrophobic surface of the substrate is now coated with hydrophilic groups of the wetting agent, making it susceptible to subsequent wetting by a formulation.

Wetting agents also include surfactants. These are negatively charged such as SDS and the like. They can also be positively charged such as cetyl pyridinium chloride (CPC), TMAC, benzalkonium chloride or neutral, such as tweens (particularly tween 20 and tween 80), sorbitans, or laureths. These wetting agents exhibit their maximum effect at and above the critical micelle concentration (CMC), and the effect is noticeable at concentrations as low as about one order of magnitude below the CMC. Wetting agents also include polymers having amphiphilic properties. These include cellulose derivatives such as HEC, HPC, HPMC, MC, HEMC, EHEC and Pluronics. These amphiphilic polymers can also be use to alter to viscosity of a solution which also effects the wettability of that solution. It is noteworthy that some proteins and peptides present wetting properties in solution that can be further enhanced by including surfactants in the solution.

### Wetting Agents in the Drug Solution

In addition to pretreatment of the microprojection with wetting agents, the wetting agent can be incorporated in the drug formulation used to coat the microprojections. This approach is particularly useful with polysaccharide drugs such as pentosan polysulfate or small molecular weight heparin, nucleic acid derivatives such as plasmid DNA or oligonucleotides and small hydrophilic molecular weight drugs such as nicotine or fentanyl. In addition, even when utilizing polypeptides that present some wetting properties, addition of wetting agents in the drug formulation is beneficial.

The method of the present invention may be utilized in the manufacture of a device for delivering through the stratum corneum, a beneficial agent which has been coated on a plurality of microprotrusions by applying to the microprotrusions a solution of the beneficial agent and a wetting agent, which is then dried to form the coating. Optionally the microprotrusions are surface treated to enhance the uniformity of the coating this is formed on the microprotrusions. The device comprises a member having a plurality, and preferably a muniplicity, of stratum corneum-piercing microprotrusions. Each of the microprotrusions has a length of less than 500 µm, or if longer than 500 µm, then means are provided to ensure that the microprotrusions penetrate the skin to a depth of no more than 500 µm. These microprotrusions have a dry coating thereon. The coating, before drying, comprises an aqueous solution of a pharmacologically active agent and a wetting agent. The pharmacologically active agent is sufficiently potent to be pharmaceutically effective in a dose that can be reasonably applied or coated o the microprotrusions. The solution, once coated onto the surfaces of the microprotrusions, provides a pharmaceutically effective amount of the pharmacologically active agent. The coating is further dried onto the microprotrusions using drying methods known in the art.

A preferred embodiment of this invention consists of a method of making a device for transdermally delivering a pharmacologically active agent. The method comprises providing a member having a plurality of stratum corneum-piercing microprotrusions. An aqueous solution of the pharmacologically active agent plus a wetting agent is applied to the microprotrusions and then dried to form a dry agent-containing coating thereon. The pharmacologically active agent is sufficiently potent to be pharmaceutically effective in a doses that can be contained within the coatings. The composition can be prepared at any temperature as long as the pharmacologically active agent is not rendered inactive due to the conditions. The solution, once coated onto the surfaces of the microprotrusions, provides a pharmaceutically effective amount of the pharmacologically active agent.

The coating thickness is preferably less than the thickness of the microprotrusions, more preferably the thickness is less than 50 µm and most preferably less than 25 µm. Generally, the coating thickness is an average thickness measured over the microprotrusions.

The most preferred agents are selected from the group consisting of ACTH (1-24), calcitonin, desmopressin, LHRH, LHRH analogs, goserelin, leuprolide, parathyroid hormone (PTH), vasopressin, deamino [Val4, D-Arg8] arginine vasopressin, buserelin, triptorelin, interferon alpha, interferon beta, interferon gamma, FSH, EPO, GM-CSF, G-CSF, IL-10, glucagon, growth hormone releasing factor (GRF) and analogs of these agents including pharmaceutically acceptable salts thereof. Preferred agents further include conventional vaccines as well as DNA vaccines and small molecular weight potent drugs such as fentanyl, sufentanil and remifentanil.

The coating can be applied to the microprotrusions using known coating methods. For example, the microprotrusions can be immersed or partially immersed into an aqueous coating solution of the agent as described in pending United States application Serial Number 10/099604, filed March 15, 2002. Alternatively the coating solution can be sprayed onto the microprotrusions. Preferably the spray has a droplet size of about 10-200 picoliters. More preferably the droplet size and placement is precisely controlled using printing techniques so that the coating solution is deposited directly onto the microprotrusions and not onto other "non-piercing" portions of the member having the microprotrusions.

Optionally, the stratum corneum-piercing microprotrusions are formed from a sheet wherein the microprotrusions are formed by etching or punching the sheet and then the microprotrusions are folded or bent out of a plane of the sheet. While the pharmacologically active agent coating can be applied to the sheet before formation of the microprotrusions, preferably the coating is applied after the microprotrusions are cut or etched out but prior to being folded out of the plane of the sheet. More preferred is coating after the microprotrusions have been folded or bent from the plane of the sheet.

### BRIEF DESCRIPTION OF THE DRAWINGS

The invention will now be described in greater detail with reference to the accompanying drawings and figures. wherein:
FIG. 1 is a perspective view of a portion of one example of a microprotrusion array; and
FIG. 2 is a perspective view of the microprotrusion array of FIG. 1 with a coating deposited onto the microprotrusions.

### MODES FOR CARRYING OUT THE INVENTION

**DEFINITIONS:**

Unless stated otherwise the following terms used herein have the following meanings.

The term "transdermal" means the delivery of an agent into and/or through the skin for local or systemic therapy.

The term "transdermal flux" means the rate of transdermal delivery.

The term "co-delivering" as used herein means that a supplemental agent(s) is administered transdermally either before the agent is delivered, before and during transdermal flux of the agent, during transdermal flux of the agent, during and after transdermal flux of the agent, and/or after transdermal flux of the agent. Additionally, two or more beneficial agents may be coated onto the microprotrusions resulting in co-delivery of the beneficial agents.

The term "pharmacologically active agent" as used herein refers to a composition of matter or mixture containing a drug which is pharmacologically effective when administered in a therapeutically effective amount. Examples of such active agents include, without limitation, leutinizing hormone releasing hormone (LHRH), LHRH analogs (such as goserelin, leuprolide, buserelin, triptorelin, gonadorelin, and napfarelin, menotropins (urofollitropin (FSH) and LH)), vasopressin, desmopressin, corticotropin (ACTH), ACTH analogs such as ACTH (1-24), calcitonin, parathyroid hormone (PTH), vasopressin, deamino [Val4, D-Arg8] arginine vasopressin, interferon alpha, interferon beta, interferon gamma, erythropoietin (EPO), granulocyte macrophage colony stimulating factor (GM-CSF), granulocyte colony stimulating factor (G-CSF), inteneukin-10 (IL-10) and glucagon. It is to be understood that more than one agent may be incorporated into the agent formulation in the method of this invention, and that the use of the term "pharmacologically active agent" in no way excludes the use of two or more such agents or drugs. The agents can be in various forms, such as free bases, acids, charged or uncharged molecules, components of molecular complexes or nonirritating, pharmacologically acceptable salts. Also, simple derivatives of the agents (such as ethers, esters, amides, etc) which are easily hydrolyzed at body pH, enzymes, etc., can be employed.

The term "therapeutically effective amount" or "therapeutically effective rate" refers to the amount or rate of the pharmacologically active agent needed to effect the desired therapeutic, often beneficial, result. The amount of agent employed in the coatings will be that amount necessary to deliver a therapeutically effective amount of the agent to achieve the desired therapeutic result. In practice, this will vary widely depending upon the particular pharmacologically active agent being delivered, the site of delivery, the severity of the condition being treated, the desired therapeutic effect and the dissolution and release kinetics for delivery of the agent from the coating into skin tissues. It is not practical to define a precise range for the therapeutically effective amount of the pharmacologically active agent incorporated into the microprotrusions and delivered transdermally according to the methods described herein.

The term "microprotrusions" refers to piercing elements which are adapted to pierce or cut through the stratum corneum into the underlaying epidermis layer, or epidermis and dermis layers, of the skin of a living animal, particularly a mammal and more particularly a human. The piercing elements should not pierce the skin to a depth which causes bleeding. Typically the piercing elements have a blade length of less than 500 microns, and preferably less than 250 microns. The microprotrusions typically have a width and thickness of about 5 to 50 microns. The microprotrusions may be formed in different shapes, such as needles, hollow needles, blades, pins, punches, and combinations thereof.

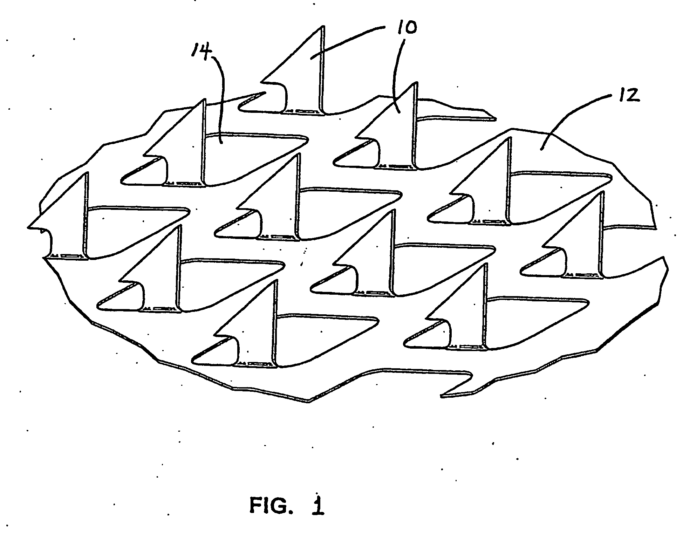

The term "microprotrusion array" as used herein refers to a plurality of microprotrusions arranged in an array for piercing the stratum corneum. The microprotrusion array may be formed by etching or punching a plurality of microprotrusions from a thin sheet and folding or bending the microprotrusions out of the plane of the sheet to form a configuration such as that shown in FIG. 1. The microprotrusion array may also be formed in other known manners, such as by forming one or more strips having microprotrusions along an edge of each of the strip(s) as disclosed in Zuck, US Patent No. 6,050,988. The microprotrusion array may include hollow needles which hold a dry pharmacologically active agent.

References to the area of the sheet or member and reference to some property per area of the sheet or member, are referring to the area bounded by the outer circumference or border of the sheet.

The term "pattern coating" refers to coating an agent onto selected areas of the microprotrusions. More than one agent may be pattern coated onto a single microprotrusion array. Pattern coatings can be applied to the microprotrusions using known micro-fluid dispensing techniques such as micropipeting and ink jet coating.

### DETAILED DESCRIPTION

There is described herein a device for transdermally delivering a pharmacologically active agent to a patient in need thereof. The device has a plurality of stratum corneum-piercing microprotrusions extending therefrom. The microprotrusions are adapted to pierce through the stratum corneum into the underlying epidermis layer, or epidermis and dermis layers, but do not penetrate so deep as to reach the capillary beds and cause significant bleeding. The microprotrusions have a dry coating thereon which contains the pharmacologically active agent. Upon piercing the stratum corneum layer of the skin, the agent-containing coating is dissolved by body fluid (Intracellular fluids and extracellular fluids such as interstitial fluid) and released into the skin for local or systemic therapy.

The kinetics of the agent-containing coating dissolution and release will depend on many factors including the nature of the drug, the coating process, the coating thickness and the coating composition (e.g., the presence of coating formulation additives). Depending on the release kinetics profile, it may be necessary to maintain the coated microprotrusions in piercing relation with the skin for extended periods of time (e.g., up to about 8 hours). This can be accomplished by anchoring the microprotrusion member to the skin using adhesives or by using anchored microprotrusions such as described in WO 97/48440.

FIG. 1 illustrates one example of a stratum corneum-piercing microprotrusion member for use in a method of the present invention. Fig. 1 shows a portion of the member having a plurality of Microprotrusions 10. The Microprotrusions 10 extend at substantially a 90° angle from Sheet 12 having Openings 14. Sheet 12 may be incorporated into a delivery patch including a backing for Sheet 12 and may additionally include adhesive for adhering the patch to the skin. In this embodiment the microprotrusions are formed by etching or punching a plurality of Microprotrusions 10 from a thin metal Sheet 12 and bending Microprotrusions 10 out of the plane of the sheet Metals such as stainless steel and titanium are preferred. Metal microprotrusion members are disclosed In Trautman et al. U.S. Patent 6,083,196; Zuck, U.S. Patient 6,050,988; and Daddona et al., U.S. Patent 6,091,975.

Other microprotrusion members that can be used with the present invention are formed by etching silicon using silicon chip etching techniques or by molding plastic using etched micro-molds. Silicon and plastic microprotrusion members are disclosed in Godshall et al., U.S. Patent 5,879,326.

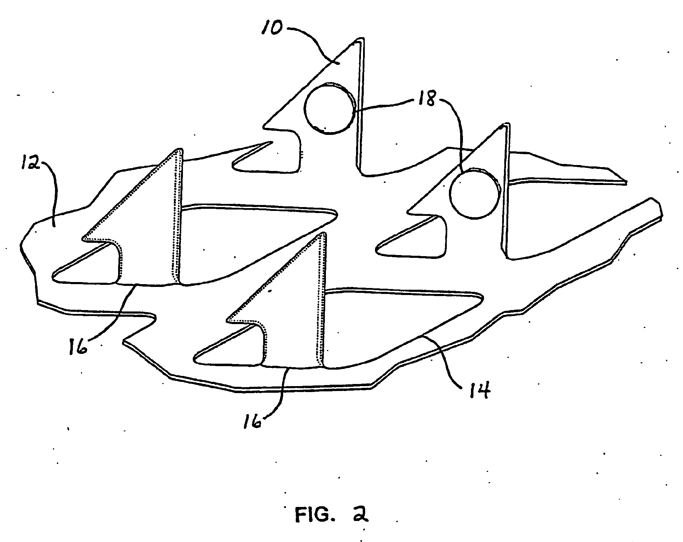

FIG. 2 illustrates the microprotrusion member having Micrcprotrusions 10 having a pharmacologically active agent-containing Coating 16. Coating 16 may partially or completely cover the Microprotrusion 10. For example, the coating can be in a dry pattern coating on the microprotrusions. The coatings can be applied before or after the microprotrusions are formed.

The coating on the microprotrusions can be formed by a variety of known methods. One such method is dip-coating. Dip-coating can be described as a means to coat the microprotrusions by partially or totally immersing the microprotrusions into the drug-containing coating solution. Alternatively the entire device can be immersed into the coating solution. Coating only those portions the microprotrusion member which pierce the skin is preferred.

By use of the partial immersion technique described above, it is possible to limit the coating to only the tips of the microprotrusions. There is also a roller coating mechanism that limits the coating to the tips of the microprotrusion. This technique is described in a United States patent application (serial number 10/099.604) filed 16 March 2001.

Other coating methods include spraying the coating solution onto the microprotrusions. Spraying can encompass formation of an aerosol suspension of the coating composition. In a preferred embodiment an aerosol suspension forming a droplet size of about 10 to 200 picoliters is sprayed onto the microprotrusions and then dried. In another embodiment, a very small quantity of the coating solution can be deposited onto the Microprotrusions 10 as shown in Fig. 2 as Pattern Coating 18. The Pattern Coating 18 can be applied using a dispensing system for positioning the deposited liquid onto the microprotrusion surface. The quantity of the deposited liquid is preferably in the range of 0.5 to 20 nanoliters/microprotrusion. Examples of suitable precision metered liquid dispensers are disclosed in US Patent Nos. 5,916,524; 5,743,960; 5,741,554; and 5,738,728.

Microprotrusion coating solutions can also be applied using ink jet technology using known solenoid valve dispensers, optional fluid motive means and positioning means which is generally controlled by use of an electric field. Other liquid dispensing technology from the printing industry or similar liquid dispensing technology known in the art can be used for applying the pattern coating of this invention.

The coating solutions used in the present invention are solutions or suspensions of the pharmacologically active agent and optionally a wetting agent. The solution must have a viscosity of less than about 200 centipoise and greater than 3 centipoise In order to effectively coat the microprotrusion properly. The viscosity of the coating solution can be adjusted by changing the drug concentration of the formulation or by addition of a viscosity enhancing agent such as cellulose derivatives or increasing the solid content with excipients such as sucrose, trehalose, melezitose, sorbitol, mannitol and the like.

The desired coating thickness is dependent upon the density of the microprotrusions per unit area of the sheet and the viscosity and concentration of the coating composition as well as the coating method chosen. In general, coating thickness should be less than 50 microns since thicker coatings have a tendency to slough off the microprotrusions upon stratum corneum piercing. A preferred coating thickness is less than 10 microns as measured from the microprotrusion surface. Generally coating thickness is referred to as an average coating thickness measured over the coated microprotrusion. A more preferred coating thickness is about 1 to 10 microns.

The agents used in the present invention require a dose of about 10 micrograms to about 2 milligrams. Amounts within this range can be coated onto a microprotrusion array of the type shown in FIG. 1 having the Sheet 12 with an area of up to 10 cm² and a microprotrusion density of up to 1000 microprotrusions per cm².

Preferred pharmacologically active agents having the properties described above are selected from the group consisting of desmopressin, luteinizing hormone releasing hormone (LHRH) and LHRH analogs (e.g., goserelin, leuprolide, buserelin, triptorelin), PTH, calcitonin, vasopressin, deamino [Val4, D-Arg8] arginine vasopressin, interferon alpha, interferon beta, interferon gamma, menotropins (urofollotropin (FSH) and leutinizing hormone (LH), erythrepoietrin (EPO), GM-CSF, G-CSF, IL-10, GRF, conventional vaccines, DNA vaccines and glucagon.

In all cases, after a coating has been applied, the coating solution is dried onto the microprotrusions by various means. In a preferred embodiment the coated device is dried in ambient room conditions. However, various temperatures and humidity levels can be used to dry the coating solution onto the microprotrusions. Additionally, the devices can be heated, lyophilized, freeze dried or similar techniques used to remove the water from the coating.

Other known formulation adjuvants can be added to the coating solution as long as they do not adversely affect the necessary solubility and viscosity characteristics of the coating solution and the physical integrity of the dried coating.

The following examples are given to enable those skilled in the art to more clearly understand and practice the present invention. They should not be considered as limiting the scope of the invention but merely as being illustrated as representative thereof.

### Example 1

As an example of the method of pretreatment of a microprojection with a wetting agent, the following test was performed.

Pentosan polysulfate (PPS) was used as the model drug, which has poor wetting properties. A 20 wt% PPS solution was prepared in water. Fluorescein, was also included in this solution at a concentration of 0.001 M. The fluorescein was included to aid in the visual microscopic evaluation of the coatings that were formed.

A strip of titanium foil was first cleaned with acetone and then dipped into a 0.1% solution of sodium dodecyl sulfate (SDS). The strip was washed with water and dried by blotting. The strip was subsequently dipped in the PPS solution and left to dry for 1 hour at room temperature. Additional untreated and pre-etched titanium strips were also dipped in the PPS solution and dried. Evaluation was made by visually examining the strips under a fluorescence microscope. Results indicated that pretreatment of the titanium foil strip with wetting agents improved the homogeneity of the coating when compared to the untreated or pre-etched material.

### Example 2

### Drugs With Poor Wetting Characteristics.

Pentosan polysulfate (PPS) was used as the model drug with poor wetting properties. A 20 wt% PPS solution was prepared in water. To this solution, various wetting agents were added at different concentrations. In all solutions, fluorescein was also present at 0.001 M for evaluation of the coating. A strip of titanium foil cleaned with acetone was dipped in a solution and left to dry for 1 hour at room temperature. Evaluation of the coating was performed visually by fluorescence microscopy. The coating that resulted from each test formulation was rated as either poor, fair, or good. Results indicate that wetting agents improve the homogeneity of the coating (Table 1). In addition, microscopy revealed that an amorphous glassy material was obtained upon drying. Dissolution of the mixture following rehydration was very fast.

**Table 1: Effect of Wetting Agents on Coating Homogeneity of a 20 % PPS Solution**

| Wetting Agent | Concentration (%) | Coating homogeneity |
|---|---|---|
| None | - | Poor |
| SDS | 0.1 | Good |
| SDS | 0.01 | Good |
| SDS | 0.001 | Poor |
| Tween 80 | 1 | Good |
| Tween 80 | 0.1 | Good |
| Tween 80 | 0.01 | Poor |
| HEC | 0.1 | Good |
| HEC | 0.01 | Poor |

### Example 3

### Drugs At Low Concentration Included In A Carrier Matrix With Poor Wetting Characteristics.

Melezitose (a trisaccharide, composed of two molecules of glucose and one of fructose, molecular weight of 504.44) was used as the model carrier and ovalbumin as the model drug. A 20 wt% Melezitose, 0.1 wt% ovalbumin solution was prepared in water. To this solution, various wetting agents were added at different concentrations. In all solutions, fluorescein was also present at 0.001 M for evaluation of the coating. A strip of titanium foil cleaned with acetone was dipped in a solution and left to dry for 1 hour at room temperature. Evaluation was performed by fluorescence microscopy. Results indicate that wetting agents improve the homogeneity of the coating (Table 2). In addition, microscopy revealed that an amorphous glassy material was obtained upon drying. Dissolution of the mixture following rehydration was very fast.

**Table 2: Effect of Wetting Agents on Coating Homogeneity of a 20% Melezitose, 1% Ovalbumin Solution**

| Additive | Concentration (%) | Coating homogeneity |
|---|---|---|
| None | - | Poor |
| SDS | 0.1 | Good |
| SDS | 0.01 | Good |
| SDS | 0.001 | Poor |
| Tween 80 | 1 | Good |
| Tween 80 | 0.1 | Good |
| Tween 80 | 0.01 | Poor |
| HEC | 0.1 | Good |
| HEC | 0.01 | Poor |

Note that at this concentration, ovalbumin does not present good wetting characteristics. Higher concentrations of ovalbumin would not necessitate the addition of wetting agents to improve the coating properties of the formulation.

### Example 4

### Drug Particles Included In A Carrier Matrix With Poor Wetting Characteristics.

Melezitose was used as the model carrier and 2 micron diameter fluorescent beads as the model drug particles. A 20 wt% Melezitose, 2 wt% beads solution was prepared in water. To this solution, various wetting agents were added at different concentrations. In all solutions, fluorescein was also present at 0.001 M for evaluation of the coating. A strip of titanium foil cleaned with acetone was dipped in a solution and left to dry for 1 hour at room temperature. Evaluation was performed by fluorescence microscopy. Results indicate that wetting agents improve the homogeneity of the coating (Table 3). In addition, microscopy revealed that an amorphous matrix of melezitose surrounds the fluorescent particles. These particles were freed readily following rehydration.

**Table 3: Effect of Wetting Agents on Coating Homogeneity of a 20% melezitose, 2% fluorescent beads suspension**

| Additive | Concentration (%) | Coating homogeneity |
|---|---|---|
| None | - | Poor |
| SDS | 0.1 | Good |
| SDS | 0.01 | Good |
| SDS | 0.001 | Poor |
| Tween 80 | 1 | Good |
| Tween 80 | 0.1 | Good |
| Tween 80 | 0.01 | Poor |
| HEC | 0.1 | Good |
| HEC | 0.01 | Poor |

### Example 5

### Effect of Viscosity

Pentosan Polysulphate (PPS) was used as the model drug with poor wetting properties. A 45% w/w PPS solution was prepared in water. The viscosity of the formulation was evaluated and found to be 53 centipoise at a shear rate of 667s⁻¹. The contact angle of the formulation was 90°. The contact angle can be defined as the angle between the substrate support surface and the tangent line at the point of contact of the liquid droplet with the substrate. The coating was found to be fairly homogenous with a CV of about 30%. This example highlights the importance of viscosity. In this example increasing the viscosity of the solution resulted in a homogenous coating (*cf.* Table 1 Example 2 with formulation containing no wetting agent).

The table below Illustrates that by varying the viscosity, by varying the sucrose concentration, the wettability of a poorly wettable solution can be enhanced without the use of surfactants.

| Viscosity (centipoises) | Sucrose Concentration (%w/w) | Quality of ceating¹ |
|---|---|---|
| 3 | 30 | Did not coat |
| 7 | 40 | Coatable |
| 19 | 50 | Homogeneous coating |
| 61 | 60 | Homogeneous coating |
| 100 | 65 | Coatable |

| | | |
|---|---|---|
| ¹"Did not coat" indicates formulation was not coated on the microprojections. | | |
| "Coatable" indicates that there was coating on the microprojections. | | |
| "Homogeneous coating" Indicates that the coating from microprojection to microprojection and from array to array was homogenous. | | |

Although the previous examples have discussed separately the techniques of surface pretreatment and inclusion of wetting agents in the drug formulation, these two methods can be performed separately as discussed or both utilized in a single embodiment.

## Claims

1. A method of coating the surface of one or more microprojections of a microprojection array comprising the steps of :
(i) providing a microprojection array comprised of one or more microprojections;
(ii) treating the surface of one or more of said microprojections of said microprojection array with a method selected from the group consisting of chemical pre-etching, plasma treatment, heat treating, rinsing with an alkaline detergent and rinsing with a wetting agent;
(iii) providing a coating formulation comprising an active agent;
(iv) applying said coating formulation to said treated surfaces of said one or more microprojections; and
(v) drying said coating formulation onto said surfaces to form a coating.

2. The method of coating the surface of one or more microprojections of a microprojection array as disclosed in claim 1 wherein said coating formulation contains a pharmacological effective dose of said agent.

3. The method of coating the surface of one or more microprojections of a microprojection array as disclosed in claim 1 or 2; wherein when the step of treating the surface of one or more of said microprojections of said microprojection array is the method of rinsing with a wetting agent; steps (ii), (iii) and (iv) are performed simultaneously; and the coating formulation comprises the wetting agent and an active agent.

4. The method of coating the surface of one or more microprojections of a microprojection array as disclosed in claim 1 or 2 wherein said step of treating comprises chemical pre-etching.

5. The method of coating the surface of one or more microprojections of a microprojection array as disclosed in claim 1 or 2 wherein said step of treating comprises plasma treatment.

6. The method of coating the surface of one or more microprojections of a microprojection array as disclosed in claim 1 or 2 wherein said step of treating comprises heat treating.

7. The method of coating the surface of one or more microprojections of a microprojection array as disclosed in claim 1 or 2 wherein said step of treating comprises rinsing at least one surface of one or more microprojections with an alkaline detergent.

8. The method of coating the surface of one or more microprojections of a microprojection array as disclosed in claim 1, 2 or 3 wherein said step of treating comprises rinsing at least one surface of one or more microprojections with a wetting agent.

9. The method of coating the surface of one or more microprojections of a microprojection array as disclosed in claim 8 wherein said wetting agent comprises a surfactant.

10. The method of coating the surface of one or more microprojections of a microprojection array a3 disclosed in claim 9 wherein said surfactant comprises a surfactant selected from the group consisting of sodium dodecyl sulfate, cetyl pyridinium chloride, TMAC, benzalkonium chloride, tweens, sorbitans, and laureths.

11. The method of coating the surface of one or more microprojections of a microprojection array as disclosed in any one of claims 8 to 10 wherein said wetting agent is present in a concentration at or above the critical micelle concentration.

12. The method of coating the surface of one or more microprojections of a microprojection array as disclosed in claim 8 wherein said wetting agent comprises a wetting agent selected from the group consisting of HEC, HPC, HPMC, MC, HEMC, EHEC and pluronics.

13. The method of coating the surface of one or more microprojections of a microprojection array as disclosed in claim 8 wherein said wetting agent comprises a wetting agent selected from the group consisting of proteins and peptides.

14. The method of coating the surface of one or more microprojections of a microprojection array as disclosed in claim 10 wherein said tweens comprise a tween selected from the group consisting of tween 20 and tween 80.

15. The method of coating the surface of one or more microprojections of a microprojection array as disclosed in claim 1 or 3 wherein said coating formulation has a viscosity from 3 centipoise to 200 centipoise and said coating formulation has a contact angle of less than 100 degrees.

## Patentansprüche

1. Verfahren zur Beschichtung der Oberfläche eines oder mehrerer Mikrovorsprünge einer Mikrovorsprungsanordnung, umfassend die folgenden Schritte:
(i) Bereitstellen einer Mikrovorsprungsanordnung aus einem oder mehreren Mikrovorsprüngen;
(ii) Behandeln der Oberfläche von einem oder mehreren Mikrovorsprüngen der Mikrovorsprungsanordnung nach einem Verfahren, das ausgewählt wird aus der Gruppe, die aus chemischem Vorätzen, Plasmabehandlung, Wärmebehandlung, Spülen mit einem alkalischen Tensid und Spülen mit einem Benetzungsmittel besteht;
(iii) Bereitstellen einer Beschichtungsformulierung, die einen Wirkstoff umfasst;
(iv) Aufbringen der Beschichtungsformulierung auf die behandelten Oberflächen des einen bzw. der mehreren Mikrovorsprünge; und
(v) Trocknen der Beschichtungsformulierung auf den Oberflächen, um eine Beschichtung zu erhalten.

2. Verfahren zur Beschichtung der Oberfläche eines oder mehrerer Mikrovorsprünge einer Mikrovorsprungsanordnung wie in Anspruch 1 beschrieben, worin die Beschichtungsformulierung eine pharmakologisch wirksame Dosis des Mittels enthält.

3. Verfahren zur Beschichtung der Oberfläche eines oder mehrerer Mikrovorsprünge einer Mikrovorsprungsanordnung wie in Anspruch 1 oder 2 beschrieben, worin in dem Fall, wo der Schritt des Behandelns der Oberfläche von einem oder mehreren Mikrovorsprüngen der Mikrovorsprungsanordnung das Verfahren des Spülens mit einem Benetzungsmittel ist, die Schritte (ii), (iii) und (iv) gleichzeitig durchgeführt werden, und die Beschichtungsformulierung das Benetzungsmittel und einen Wirkstoff umfasst.

4. Verfahren zur Beschichtung der Oberfläche eines oder mehrerer Mikrovorsprünge einer Mikrovorsprungsanordnung wie in Anspruch 1 oder 2 beschrieben, worin der Schritt der Behandlung chemisches Vorätzen umfasst.

5. Verfahren zur Beschichtung der Oberfläche eines oder mehrerer Mikrovorsprünge einer Mikrovorsprungsanordnung wie in Anspruch 1 oder 2 beschrieben, worin der Schritt der Behandlung Plasmabehandlung umfasst.

6. Verfahren zur Beschichtung der Oberfläche eines oder mehrerer Mikrovorsprünge einer Mikrovorsprungsanordnung wie in Anspruch 1 oder 2 beschrieben, worin der Schritt der Behandlung Wärmebehandlung umfasst.

7. Verfahren zur Beschichtung der Oberfläche eines oder mehrerer Mikrovorsprünge einer Mikrovorsprungsanordnung wie in Anspruch 1 oder 2 beschrieben, worin der Schritt der Behandlung das Spülen der Oberfläche eines oder mehrerer Mikrovorsprünge mit einem alkalischen Tensid umfasst.

8. Verfahren zur Beschichtung der Oberfläche eines oder mehrerer Mikrovorsprünge einer Mikrovorsprungsanordnung wie in Anspruch 1, 2 oder 3 beschrieben, worin der Schritt der Behandlung das Spülen der Oberfläche eines oder mehrerer Mikrovorsprünge mit einem Benetzungsmittel umfasst.

9. Verfahren zur Beschichtung der Oberfläche eines oder mehrerer Mikrovorsprünge einer Mikrovorsprungsanordnung wie in Anspruch 8 beschrieben, worin das Benetzungsmittel ein Tensid umfasst.

10. Verfahren zur Beschichtung der Oberfläche eines oder mehrerer Mikrovorsprünge einer Mikrovorsprungsanordnung wie in Anspruch 9 beschrieben, worin das Tensid ein Tensid umfasst, das aus der Gruppe ausgewählt ist, die aus Natriumdodecylsulfat, Cetylpyridiniumchlorid, TMAC, Benzalkoniumchlorid, Tweenen, Sorbitanen und Laureths besteht.

11. Verfahren zur Beschichtung der Oberfläche eines oder mehrerer Mikrovorsprünge einer Mikrovorsprungsanordnung wie in einem der Ansprüche 8 bis 10 beschrieben, worin das Benetzungsmittel in einer Konzentration bei oder oberhalb der kritischen Mizellenkonzentration vorliegt.

12. Verfahren zur Beschichtung der Oberfläche eines oder mehrerer Mikrovorsprünge einer Mikrovorsprungsanordnung wie in Anspruch 8 beschrieben, worin das Benetzungsmittel ein Benetzungsmittel umfasst, das aus der Gruppe ausgewählt ist, die aus HEC, HPC, HPMC, MC, HEMC, EHEC und Pluronics besteht.

13. Verfahren zur Beschichtung der Oberfläche eines oder mehrerer Mikrovorsprünge einer Mikrovorsprungsanordnung wie in Anspruch 8 beschrieben, worin das Benetzungsmittel ein Benetzungsmittel umfasst, das aus der Gruppe ausgewählt ist, die aus Proteinen und Peptiden besteht.

14. Verfahren zur Beschichtung der Oberfläche eines oder mehrerer Mikrovorsprünge einer Mikrovorsprungsanordnung wie in Anspruch 10 beschrieben, worin die Tweene ein Tween umfassen, das aus der Gruppe ausgewählt ist, die aus Tween 20 und Tween 80 besteht.

15. Verfahren zur Beschichtung der Oberfläche eines oder mehrerer Mikrovorsprünge einer Mikrovorsprungsanordnung wie in Anspruch 1 oder 3 beschrieben, worin die Beschichtungsformulierung eine Viskosität von 3 Zentipoise bis 200 Zentipoise hat, und die Beschichtungsformulierung einen Kontaktwinkel von weniger als 100 Grad hat.

## Revendications

1. Procédé de revêtement de la surface d'une ou plusieurs microprojections d'un ensemble de microprojections, comprenant les étapes consistant à :
(i) prendre un ensemble de microprojections comprenant une ou plusieurs microprojections ;
(ii) traiter la surface d'une ou plusieurs desdites microprojections dudit ensemble de microprojections par une méthode choisie dans le groupe consistant en une pré-attaque chimique, un traitement par plasma, un traitement thermique, un rinçage avec un détergent alcalin et un rinçage avec un agent mouillant ;
(iii) prendre une formulation de revêtement comprenant un agent actif ;
(iv) appliquer ladite formulation de revêtement sur lesdites surfaces traitées de la ou des microprojections précitées ; et
(v) sécher ladite formulation de revêtement sur lesdites surfaces afin de former un revêtement.

2. Procédé de revêtement de la surface d'une ou plusieurs microprojections d'un ensemble de microprojections, selon la revendication 1, dans lequel ladite formulation de revêtement contient une dose pharmacologiquement efficace dudit agent.

3. Procédé de revêtement de la surface d'une ou plusieurs microprojections d'un ensemble de microprojections, selon l'un des revendications 1 ou 2, dans lequel, lorsque l'étape de traitement de la surface d'une ou plusieurs desdites microprojections dudit ensemble de microprojections est la méthode de rinçage avec un agent mouillant, les étapes (ii), (iii) et (iv) sont réalisées simultanément, et la formulation de revêtement comprend l'agent mouillant et un agent actif.

4. Procédé de revêtement de la surface d'une ou plusieurs microprojections d'un ensemble de microprojections, selon l'une des revendications 1 ou 2, dans lequel ladite étape de traitement comprend une pré-attaque chimique.

5. Procédé de revêtement de la surface d'une ou plusieurs microprojections d'un ensemble de microprojections, selon l'une des revendications 1 ou 2, dans lequel ladite étape de traitement comprend un traitement par plasma.

6. Procédé de revêtement de la surface d'une ou plusieurs microprojections d'un ensemble de microprojections, selon l'une des revendications 1 ou 2, dans lequel ladite étape de traitement comprend un traitement thermique.

7. Procédé de revêtement de la surface d'une ou plusieurs microprojections d'un ensemble de microprojections, selon l'une des revendications 1 ou 2, dans lequel ladite étape de traitement comprend un rinçage d'au moins une surface d'une ou plusieurs microprojections par un détergent alcalin.

8. Procédé de revêtement de la surface d'une ou plusieurs microprojections d'un ensemble de microprojections, selon l'une des revendications 1, 2 ou 3, dans lequel ladite étape de traitement comprend un rinçage d'au moins une surface d'une ou plusieurs microprojections avec un agent mouillant.

9. Procédé de revêtement de la surface d'une ou plusieurs microprojections d'un ensemble de microprojections, selon la revendication 8, dans lequel ledit agent mouillant comprend un agent tensio-actif.

10. Procédé de revêtement de la surface d'une ou plusieurs microprojections d'un ensemble de microprojections, selon la revendication 9, dans lequel ledit agent tensio-actif comprend un agent tensio-actif choisi dans le groupe constitué par le dodécyl sulfate de sodium, le chlorure de cétyl pyridinium, le TMAC, le chlorure de benzalkonium, les tweens, les sorbitans et les laureths.

11. Procédé de revêtement de la surface d'une ou plusieurs microprojections d'un ensemble de microprojections, selon l'une des revendications 8 à 10, dans lequel ledit agent mouillant est présent dans une concentration à ou au-dessus de la concentration micellaire critique.

12. Procédé de revêtement de la surface d'une ou plusieurs microprojections d'un ensemble de microprojections, selon la revendication 8, dans lequel ledit agent mouillant comprend un agent mouillant choisi dans le groupe constitué par HEC, HPC, HPMC, MC, HEMC, EHEC et les pluronics.

13. Procédé de revêtement de la surface d'une ou plusieurs microprojections d'un ensemble de microprojections, selon la revendication 8, dans lequel ledit agent mouillant comprend un agent mouillant choisi dans le groupe constitué par les protéines et les peptides.

14. Procédé de revêtement de la surface d'une ou plusieurs microprojections d'un ensemble de microprojections, selon la revendication 10, dans lequel lesdits tweens comprennent un tween choisi dans le groupe constitué par le tween 20 et le tween 80.

15. Procédé de revêtement de la surface d'une ou plusieurs microprojections d'un ensemble de microprojections, selon l'une des revendications 1 ou 3, dans lequel ladite formulation de revêtement a une viscosité de 3 centipoises à 200 centipoises et ladite formulation de revêtement a un angle de contact de moins de 100 degrés.
